(19) Europäisches Patentamt / European Patent Office / Office européen des brevets

(11) EP 4 628 879 A1

(12) EUROPEAN PATENT APPLICATION
published in accordance with Art. 153(4) EPC

(43) Date of publication:
08.10.2025 Bulletin 2025/41

(21) Application number: 23910507.5

(22) Date of filing: 25.12.2023

(51) International Patent Classification (IPC):
$G01N\ 23/046$ (2018.01)    $A61B\ 6/03$ (2006.01)
$G01V\ 5/00$ (2024.01)

(86) International application number:
PCT/CN2023/141574

(87) International publication number:
WO 2024/140581 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.12.2022 CN 202211708553

(71) Applicants:
• Tsinghua University
Haidian District,
Beijing 100084 (CN)
• NUCTECH COMPANY LIMITED
Beijing 100084 (CN)

(72) Inventors:
• CHEN, Zhiqiang
Beijing 100084 (CN)
• LI, Yuanjing
Beijing 100084 (CN)
• SUN, Shangmin
Beijing 100084 (CN)
• YI, Xi
Beijing 100084 (CN)
• LIU, Bicheng
Beijing 100084 (CN)
• ZONG, Chunguang
Beijing 100084 (CN)

(74) Representative: Cabinet Beau de Loménie
103, rue de Grenelle / CS 90800
75340 Paris Cedex 07 (FR)

(54) **CT SCANNING SYSTEM AND METHOD**

(57) The present invention relates to a CT scanning system and method. The scanning system comprises: at least one ray source group, used for emitting rays to an object to be scanned, each ray source group comprising a plurality of ray sources, and the plurality of ray sources being spaced apart in the same scanning plane and having different ray spread angle directions; and at least one detector, arranged in one-to-one correspondence with the at least one ray source group, each detector receiving rays which are emitted from the corresponding ray source group and transmit through the object to be scanned. During a scanning process, the at least one ray source group rotates around the object to be scanned, so as to scan said object at a plurality of rotational angles, and at the same rotational angle, the plurality of ray sources in each ray source group emit ray beams one by one.

FIG. 2

EP 4 628 879 A1

**Description**

CROSS-REFERENCE TO RELATED APPLICATIONS

This application claims priority to Chinese Patent Application No. 202211708553.3, titled "CT SCANNING SYSTEM AND METHOD" and filed on December 28, 2022, which is hereby incorporated by reference in its entirety.

TECHNICAL FIELD

The present application relates to the field of CT imaging technology, and in particular to a CT scanning system and a CT scanning method.

BACKGROUND

CT scanning technology can measure the ray projection of objects at different angles. With appropriate imaging algorithms, it can clearly, accurately and intuitively display the internal structure, composition, material and defect status of the detected object in the form of two-dimensional or three-dimensional images. Therefore, it has not only become an indispensable and effective means for medical clinical diagnosis and analysis and determination of lesions, but also gradually become an important tool in the fields of industrial non-destructive testing, material organization analysis, cargo/vehicle safety inspection, etc.

There may be individual differences between CT systems in different application scenarios, but the basic structures are roughly the same. A typical CT scanning system consists of a ray source, a detector, a scanning and control apparatus, a data acquisition and processing apparatus, an image reconstruction and display apparatus, etc.

SUMMARY

In existing CT systems, the numbers of ray sources and detectors are often the same. The rays emitted by each ray source irradiate the object to be detected, pass through the object and are received by the corresponding detector. In such a CT scanning system, the imaging field of view is limited by the system size and the ray divergence angle. If a larger imaging field of view is obtained under complete data conditions, usually, the choice is to enlarge the divergence angle of the ray source and increase the size of the detector while keeping the overall size of the system unchanged, or to keep the divergence angle of the ray source beam unchanged and increase the distance between the ray source and the detector. The former solution may lead to a decrease in image quality due to the dose angle distribution of the ray source beam. The latter solution can avoid the decrease in image quality due to the dose angle distribution of the ray source with a large divergence angle, but it may increase the overall size of the system, which may bring inconvenience to the design, construction, radiation protection, construction and maintenance of the device.

In order to expand the imaging field of view without increasing the sizes of the detector and the system, the present application proposes a CT scanning system, which includes: at least one ray source group, configured to emit rays to a scanned object, each of the ray source groups comprising a plurality of ray sources, the plurality of ray sources being arranged at intervals from each other in a same scanning plane and having different ray divergence angle directions; and at least one detector, arranged in one-to-one correspondence with the at least one ray source group, each detector being configured to receive the rays emitted from the corresponding ray source group and transmitted through the scanned object, wherein the at least one ray source group is configured to rotate around the scanned object during scanning to scan the scanned object at a plurality of rotation angles, and at the same rotation angle, the plurality of ray sources in each of the at least one ray source group emit ray beams in sequence.

According to some embodiments, during scanning, the at least one detector rotates synchronously with the at least one ray source group.

According to some embodiments, the ray beams of the plurality of ray sources in each of the ray source groups have a same energy and/or a same divergence angle, or the ray beams of the plurality of ray sources in each of the ray source groups have different energies and/or different divergence angles.

According to some embodiments, the plurality of ray sources in each of the ray source groups are arranged at intervals along a tangent direction of a rotation direction in the same scanning plane.

According to some embodiments, the plurality of ray sources in each of the ray source groups are arranged in an arc or straight line along the tangent direction.

According to some embodiments, each of the ray source groups is formed as a multi-focus distributed ray source or includes a plurality of independent ray source modules.

According to some embodiments, each detector in the at least one detector is a single-row detector or a multi-row detector; and each detector is straight or arc-shaped.

According to some embodiments, the energies of the ray beams of the plurality of ray sources in each of the ray source groups are set to decrease in sequence from a middle to both sides of an arrangement of the plurality of ray sources.

According to some embodiments, the CT scanning system further comprises a control apparatus configured to control numbers and positions of the ray sources that emit rays in each of the ray source groups according to a size of the scanned object.

According to some embodiments, a detector crystal in each of the at least one detector is configured to be independently rotatable, so that a ray receiving surface of the detector crystal in the corresponding detector is configured to be adjusted to be perpendicular to the ray beams of each of the ray sources when the plurality of ray sources in each of the at least one ray source group emit ray beams in sequence.

According to some embodiments, the CT scanning system further comprises a control apparatus, configured to control the at least one ray source group to rotate around the scanned object so that the rays emitted by the at least one ray source group cover every point in the scanned object over a range of at least 180 degrees.

According to some embodiments, the control apparatus is configured to control the plurality of ray sources in each of the at least one ray source group to sequentially emit rays in a time-sharing on and off manner at each of the rotation angles.

According to some embodiments, the CT scanning system comprises a plurality of the ray source groups, wherein the control apparatus is configured to control the plurality of ray source groups to emit rays simultaneously at each of the rotation angles and control the plurality of ray sources in each of the plurality of ray source groups to sequentially emit rays in a time-sharing on and off manner.

According to some embodiments, the CT scanning system further comprises a data acquisition and processing apparatus, configured to receive data collected by the detector, integrate information for image reconstruction, and generate projection data, wherein the data acquisition and processing apparatus distinguishes data based on rays from different ray sources in the same ray source group by receiving time or using a marking signal.

According to some embodiments, the data acquisition and processing apparatus is configured to receive the data collected by the detector in a time-sharing acquisition manner.

According to some embodiments, the CT scanning system further comprises an image analysis and reconstruction apparatus, configured to process the projection data generated by the data acquisition and processing apparatus to generate a two-dimensional or three-dimensional image of the scanned object, wherein the image analysis and reconstruction apparatus performs an image rearrangement on the data from the plurality of ray sources in the same ray source group and performs a redundant processing on an overlapping projection data generated by the plurality of ray sources in the same ray source group before generating the two-dimensional or three-dimensional image of the scanned object.

According to some embodiments, the image analysis and reconstruction apparatus is configured to perform an image reconstruction using an analytical reconstruction algorithm or an iterative reconstruction algorithm.

The present application also provides a method for scanning the scanned object using the CT scanning system according to any of the above embodiments, the method comprising: controlling the at least one ray source group to rotate around the scanned object so that the rays emitted by the at least one ray source group cover every point in the scanned object over the range of at least 180 degrees.

According to some embodiments, the method further comprises controlling the plurality of ray sources in each of the at least one ray source group to emit rays in sequence in the time-sharing on and off manner at each of the rotation angles.

According to some embodiments, the CT scanning system comprises a plurality of the ray source groups, and the method further comprises controlling the plurality of ray source groups to emit rays simultaneously at each of the rotation angles, and controlling the plurality of ray sources in each of the plurality of ray source groups to emit rays in sequence in the time-sharing on and off manner.

According to some embodiments, the method further comprises receiving the data collected by the detector, integrating the information for image reconstruction, and generating the projection data, wherein the data based on the rays from different ray sources in the same ray source group are distinguished by receiving time or using the marking signal.

According to some embodiments, the data collected by the detector is received in the time-sharing acquisition manner.

According to some embodiments, the method further comprises processing the generated projection data to generate the two-dimensional or three-dimensional image of the scanned object, wherein before generating the two-dimensional or three-dimensional image of the scanned object, the image rearrangement is performed on data from the plurality of ray sources in the same ray source group and the redundant processing is performed on the overlapping projection data generated by the plurality of ray sources in the same ray source group.

BRIEF DESCRIPTION OF THE DRAWINGS

FIG. 1 shows a schematic diagram of a calculation of an imaging field of view of a CT scanning system in the prior art.
FIG. 2 shows a schematic structural diagram of a CT scanning system according to an embodiment of the present application.
FIG. 3 shows a schematic structural diagram of the CT scanning system with different ray source arrangements according to an embodiment of the present application.
FIG. 4 shows a comparison schematic diagram of the imaging fields of view of a CT scanning system (a) in the prior art and a CT scanning system according to an embodiment of the present application.
FIG. 5 shows a comparison schematic diagram of sizes of the CT scanning system (a) in the prior art and the CT scanning system according to an embodiment of the present application at the same ray divergence angle and imaging field of view.

DETAILED DESCRIPTION

In the following, embodiments of the present application will be described in detail with reference to the drawings. The following detailed description and drawings are used to exemplarily illustrate the principles of the present application. The schemes described in the following embodiments do not represent all schemes of the present application. Instead, these schemes are only examples of systems and methods of various aspects in the present application involved in the attached claims. The scope of the present application is defined by the claims.

In the following, unless otherwise indicated, the same reference numerals in different drawings represent the same or similar elements.

In the following description, the scanning plane refers to the plane where the cross section of the scanned object is located when the CT scanning system scans each cross section of the scanned object. The imaging field of view refers to the maximum range that the ray beams of the CT scanning system can cover in a scanning plane. In addition, the "ray divergence angle" mentioned below refers to the divergence angle of the ray beams emitted by the ray source in the scanning plane.

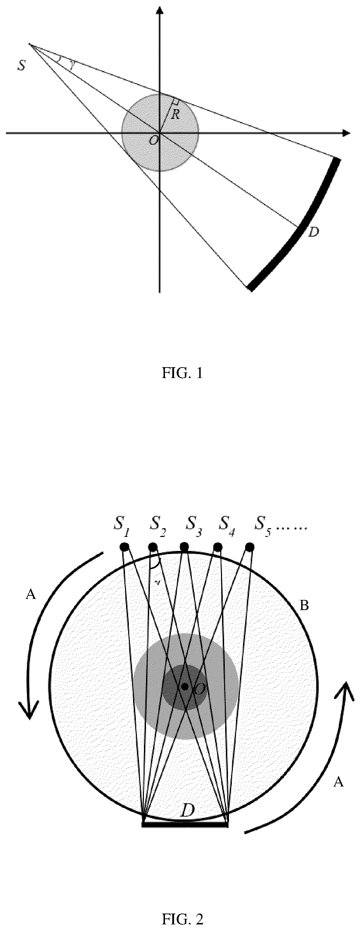

The CT scanning system of the prior art generally includes the same number of ray sources and detectors, and the ray sources and detectors are in one-to-one correspondence with each other. The rays emitted by each ray source are irradiated on the scanned object, pass through the scanned object and are received by the corresponding detector. Below, a CT scanning system including a single ray source and a single detector is taken as an example to describe the CT scanning system of the prior art. FIG. 1 discloses a schematic diagram of a CT system according to the prior art, wherein S is a ray source, D is a detector, and ray beams emitted by the ray source S (with a divergence angle of $2\gamma$ in the scanning plane, the scanning plane is the plane where the paper surface in FIG. 1 is located) is received by the detector D after passing through the scanned object (not shown in the figure). In the process of scanning the scanned object, the ray source S and the detector D can rotate synchronously around the rotation center O to generate scanning data at multiple angles. Thus, the CT scanning system can have an imaging field of view as shown in the gray shaded part in FIG. 1, the center of the imaging field of view is the rotation center O, and R is the radius of the imaging field of view, i.e., the maximum radius of the part of the scanned object that can be covered by the ray of the CT scanning system in the scanning plane. It is easy to obtain from the geometric relationship shown in FIG. 1 that the imaging field of view radius R of the CT scanning system is: $R=SO\cdot\sin(\gamma)$, wherein SO is the distance from the ray source S to the center O of the imaging field of view.

It can be seen from the above formula that if the larger size of objects need to be scanned, R needs to be increased, which can usually be achieved through two solutions: one is to keep SO unchanged and increase $\gamma$, that is, under the condition that the overall size of the system remains unchanged, increase the divergence angle of the ray beam, and at this time, the size of the detector needs to be increased simultaneously so that the ray beams can completely cover the scanned object and can be detected by the detector; the second solution is to keep $\gamma$ unchanged and increase SO, that is, keep the ray divergence angle of the ray source unchanged, and increase the distance from the ray source to the center of the imaging field of view to achieve the purpose of increasing the imaging field of view, which may obviously increase the overall size of the system.

When performing X-ray ray imaging on objects with larger sizes (for example, an outer diameter greater than 1 meter), higher energy rays (the ray energy is usually above 1MeV, such as 3MeV, 7MeV, 9MeV or up to more than 10MeV) are required to penetrate the object and obtain its internal information. However, the higher the ray energy is, the more significant the influence of the ray dose angular distribution is. The ray dose angular distribution refers to the fact that when the divergence angle of the ray beams of the ray source is relatively large, the ray dose in the edge area of the divergence angle is attenuated more than that in the center area. The part of the object irradiated by these rays in the edge area may have poor image quality due to too small ray dose. For example, the uniformity of the X-ray beams at the 7.5° divergence angle position of a 9MeV electron linear accelerator is only 55% of that at the same distance of 0°. After the rays in these areas are irradiated to the object, the rays may not penetrate the object, or after penetration, the rays may be annihilated in the background noise of the detector due to too small dose, resulting in poor quality of the final reconstructed image. Therefore, the above-mentioned first solution (increasing the ray divergence angle) is not suitable for CT scanning systems with high-energy rays. The second solution increases the distance from the ray source to the object without changing the ray divergence angle, which can avoid the degradation of image quality caused by the angular distribution of ray source dose, but increases the distance from the ray source to the center of the imaging field of view, resulting in an increase in the overall size of the system, which brings inconvenience to the design, construction, ray protection, construction and maintenance of the equipment. Therefore, both of the above solutions are not suitable for high-energy and large-field CT imaging.

In order to more conveniently scan larger objects, the present application proposes a large-field CT scanning system suitable for using high-energy rays, which can expand the imaging field of view without increasing the sizes of the detector and the system. Specifically, the CT scanning system according to the embodiments of the present application includes: at least one ray source group for emitting rays to the scanned object, each ray source group includes multiple ray sources, the multiple ray sources are spaced from one another in the same scanning plane and have different ray divergence angle directions; and at least one detector is arranged in one-to-one correspondence with at least one ray source group, each detector receives rays emitted from multiple ray sources of the corresponding ray source group and transmitted through the scanned object, wherein at least one ray source group is configured to rotate around the scanned object during the scanning process to scan the scanned object at multiple rotation angles, and at the same rotation angle, multiple ray sources in each ray source group emit ray beams in sequence. By making multiple ray sources in each ray source group emit ray beams in sequence at the same rotation angle, the imaging fields of view of multiple ray sources in the same ray source group can be spliced with one another, so that the overall irradiation area of the system can be expanded, and a large field of view scanning of larger objects can be achieved.

The CT scanning system according to an embodiments of the present application is described in detail with reference to the drawings. FIG. 2 shows a schematic diagram of a CT scanning system according to an embodiment of the present application. As shown in FIG. 2, the CT scanning system includes a ray source group S for emitting rays to a scanned object and a detector D for receiving rays emitted by the ray source group S and transmitted through the scanned object, wherein the scanned object is placed between the ray source group S and the detector D via a carrying apparatus (not shown in the figure).

The ray source group S includes a plurality of ray sources $S_1$, $S_2$, $S_3$, $S_4$, and $S_5$ for emitting rays, wherein the plurality of ray sources $S_1$, $S_2$, $S_3$, $S_4$, and $S_5$ are spaced apart from one another in the same scanning plane but have different divergence angle directions of the ray beams. Here, the scanning plane is a plane consistent with the paper surface in FIG. 2. In addition, the ray beams of each ray source $S_1$, $S_2$, $S_3$, $S_4$, and $S_5$ have the same divergence angle direction $\gamma$ and the same energy. The detector D is arranged opposite to the plurality of ray sources $S_1$, $S_2$, $S_3$, $S_4$, and $S_5$ in the scanning plane, wherein the divergence angle direction of the ray beams of each ray source $S_1$, $S_2$, $S_3$, $S_4$, and $S_5$ is set so that the ray beams of each ray source are in correspondence to the detector D. Here, the so-called "in correspondence to" generally means that the projection of the rays emitted by each ray source $S_1$, $S_2$, $S_3$, $S_4$, and $S_5$ to the detector falls within the range of the detector length $L_D$. Preferably, as shown in FIG. 2, the projection of each ray source is equal to the detector length $L_D$. By setting the divergence angle direction of the ray source in this way, it is possible to achieve ray coverage of every point in the scanned object when the ray source rotates by a relatively small angle.

During the scanning process, the ray source group S and the corresponding detector D rotate around its rotation center O to scan the scanned object at multiple rotation angles and obtain corresponding projection data. In particular, at the same rotation angle, the multiple ray sources $S_1$, $S_2$, $S_3$, $S_4$, and $S_5$ of the ray source group S sequentially emit ray beams to irradiate the scanned object.

Thus, the CT scanning system shown in FIG. 2 has an imaging field of view with a center of O (i.e., the above-mentioned rotation center O) and a radius of R in the scanning plane. Since multiple ray sources $S_1$, $S_2$, $S_3$, $S_4$, and $S_5$ are located in the same scanning plane, the imaging fields of view of respective ray sources can be spliced together to form an imaging field of view larger than the imaging field of view (for example, the dark shaded part in FIG. 2) of any single ray source, and the distance between the ray source and the detector and the size of the detector itself do not need to be changed. Therefore, the CT scanning system according to the above embodiments can expand the imaging field of view without increasing the sizes of the system and the detector.

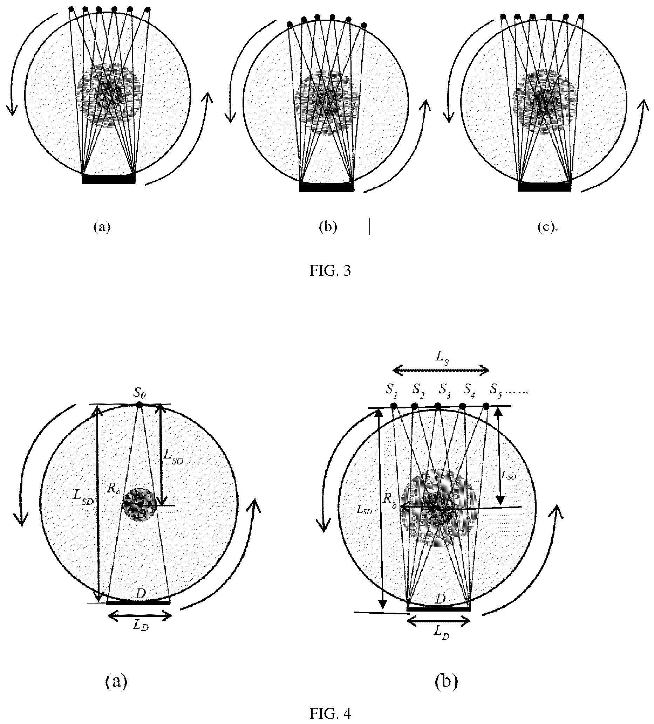

In addition, as shown in FIG. 2, the multiple ray sources $S_1$, $S_2$, $S_3$, $S_4$, and $S_5$ of the ray source group S are arranged at intervals in the scanning plane along the tangent direction of the rotation direction (as shown in A in FIG. 2). Here, the rotation direction refers to the rotation direction of the ray source group S and the detector D around the rotation center O. Specifically, the multiple ray sources of the ray source group S can be arranged in a straight line in the scanning plane along the tangent direction of the rotation direction (as shown in FIG. 2 and FIGS. 3(a) and 3(c)) or can be arranged in an arc shape (as shown in FIG. 3(b)). In some embodiments, multiple ray sources $S_1$, $S_2$, $S_3$, $S_4$, and $S_5$ are arranged on an arc-shaped slide rail, and each ray source is arranged to be movable along the slide rail within a preset range, preferably to move rapidly (for example, controlled by the control apparatus described below), so that the imaging field of view can be controlled (expanded or reduced) by controlling the spacing distances of the respective ray sources under the condition of a specified number of ray sources. It should be clear that the spacing distances of the multiple ray sources of the ray source group S should be set so that the rays emitted from the multiple ray sources can cover the entire range of the desired imaging field of view, and it is necessary to avoid the situation where part of the imaging field of view cannot be covered due to excessive spacing.

According to other embodiments, the multiple ray sources of the ray source group S may also be arranged at intervals in other directions, for example, in a direction perpendicular to the rotation plane of the ray source group S, as long as the imaging field of view can be expanded relative to any single ray source in the same scanning plane.

In addition, the multiple ray sources of the ray source group S may be formed by ray sources of a distributed multi-focus structure (which have the same number of focuses as the number of ray sources in the group), or may be multiple independent ray source modules (the number of modules is equal to the number of ray sources in the ray source S). In addition, the ray beams emitted by each ray source may be fan beams or cone beams.

In addition, as shown in FIG. 2, the ray source group S includes an odd number of ray sources, but the present application is not limited thereto. According to other embodiments, the ray source group S may include an even number of ray sources (as shown in FIG. 3). Moreover, in the embodiment of FIG. 2, the ray source group S includes 5 ray sources. However, the present application is not limited thereto. According to other embodiments, the ray source group S may include more or fewer ray sources, as long as the number of ray sources is greater than or equal to 2. In addition, the multiple ray sources in the ray source group S can be arranged symmetrically as shown in FIG. 2, or can be arranged asymmetrically (as shown in (c) in FIG. 3).

In addition, in the embodiment shown in FIG. 2, the ray divergence angles and energies of the multiple ray sources $S_1$, $S_2$, $S_3$, $S_4$, and $S_5$ in the ray source group S are the same, so as to simplify the calculation required for processing data for image reconstruction. However, the present application is not limited thereto. According to other embodiments, each of the ray divergence angles and/or energies of the multiple ray sources $S_1$, $S_2$, $S_3$, $S_4$, and $S_5$ can be different. In some embodiments, the energies of the ray beams of the multiple ray sources $S_1$, $S_2$, $S_3$, $S_4$, and $S_5$ in the ray source group S can be reduced in sequence from the middle ray source to the ray sources on both sides in the arrangement of the multiple ray sources (for example, the magnitudes of energy satisfy $S_3 > S_2 = S_4 > S_1 = S_3$), so that when using the ray sources $S_1$, $S_2$, $S_4$, and $S_5$ with lower energy, the adverse effects caused by the uneven angular distribution of the ray dose of the high-energy ray source S3 can be eliminated or reduced. Each of the ray divergence angles of the multiple ray sources $S_1$, $S_2$, $S_3$, $S_4$, and $S_5$ is set to at least cover the entire ray receiving surface of the corresponding detector to improve the detection efficiency.

In the embodiments shown in FIGS. 2 and 3, the arrangement of the detector crystals in the detector D is linear, but according to other embodiments, the arrangement of the detector crystals can also be arc-shaped. In addition, the detector D can be a single-row detector or a multi-row detector. In addition, each detector crystal in the detector D can be independently rotatably configured so that when any ray source in the ray source group S emits ray beams, each detector crystal of the detector D can adjust its respective detection surface in real time to be perpendicular to the received ray beam. In other words, when the multiple ray sources in the ray source group S emit ray beams in sequence, each detector crystal is also deflected accordingly, so that when any ray source in the ray source group S emits ray beams, the detection surface of the detector crystal is directly opposite to the any ray source.

In addition, as mentioned above, during the scanning process, the ray source group S and the detector D rotate around the rotation center O to scan the scanned object at multiple rotation angles. In order to realize the rotation of the ray source group S and the detector D, the CT scanning system may include a rotating support apparatus, such as part B in FIG. 2. The rotating support apparatus B may be a slip ring, a swivel or a bracket. The ray source group S and the detector D may rotate around the scanned object through the rotating support apparatus B, so that the rays of the ray source group S cover every point in the scanned object over a range of at least 180 degrees. The ray source group S may rotate by reciprocating rotation or continuous rotation in a single direction. In addition, the scanning trajectory of the ray source group S may be circular, spiral, linear, saddle-shaped or other scanning trajectories.

In addition, in the embodiment shown in FIG. 2, the detector D rotates synchronously with the ray source group S during the scanning process, which can minimize the size of the detector D to the greatest extent. However, the present application is not limited to this. According to other embodiments, during the scanning process, only the ray source group S rotates, and the detector D does not rotate.

Below, the advantages of the present application are explained by comparing the sizes of imaging fields of view of the CT scanning system in the prior art and the CT scanning system according to the embodiments of the present application in detail. For the convenience of explanation, the CT scanning system in the prior art including a single ray source and a single detector and the CT scanning system shown in FIG. 2 are compared as examples.

FIG. 4 (a) shows a schematic diagram of a CT scanning system in the prior art. As shown in FIG. 4 (a), the focus of the ray source of the CT scanning system is $S_0$, the detector is D, and the detector length is $L_D$. The ray source and the detector can rotate synchronously around the rotation center O to collect projection data at different rotation angles. The distance from the ray source focus $S_0$ to the rotation center O is $L_{SO}$, and the distance to the center of the detector is $L_{SD}$. The gray circular area in FIG. 4 (a) is the imaging field of view of the CT scanning system. According to the geometric relationship in FIG. 4 (a), the radius $R_a$ of the imaging field of view is:

$$R_a = \frac{L_{SO} \cdot (L_D/2)}{\sqrt{L_{SD}^2 + (L_D/2)^2}} \qquad (1)$$

In the CT scanning system according to the above embodiments of the present application, that the total length of the ray source is equal to the length of the detector is taken for illustration. That is, when $L_S = L_D$, according to the geometric relationship in FIG. 4 (b), the radius of imaging field of view is:

$$R_b = \frac{L_{SO} \cdot L_D}{L_{SD}} \qquad (2)$$

In combination with the above formula (1), it can be obtained:

$$\frac{R_b}{R_a} = \left(\frac{L_{SO} \cdot L_D}{L_{SD}}\right) \Big/ \left(\frac{L_{SO} \cdot (L_D/2)}{\sqrt{L_{SD}^2 + (L_D/2)^2}}\right) = 2 \cdot \frac{\sqrt{L_{SD}^2 + (L_D/2)^2}}{L_{SD}} > 2$$

From the above formula, it can be seen that $R_b > 2 \cdot R_a$. That is to say, the CT scanning system according to the embodiments of the present application, which adopts the arrangement of multiple ray sources corresponding to a single detector, has the radius of the imaging field of view that is more than twice that of the existing CT scanning system with a single ray source corresponding to a single detector, while the overall size of the system and the size of the detector can remain unchanged. It can be seen that the CT scanning system according to the embodiments of the present application can expand the imaging field of view without increasing the sizes of the detector and the system.

In addition, from another perspective, the CT scanning system of the above embodiments of the present application can reduce the overall size of the system and the size of the detector while keeping the ray source divergence angle and the imaging field of view unchanged compared with the above CT scanning system in the prior art. The specific comparison is as follows.

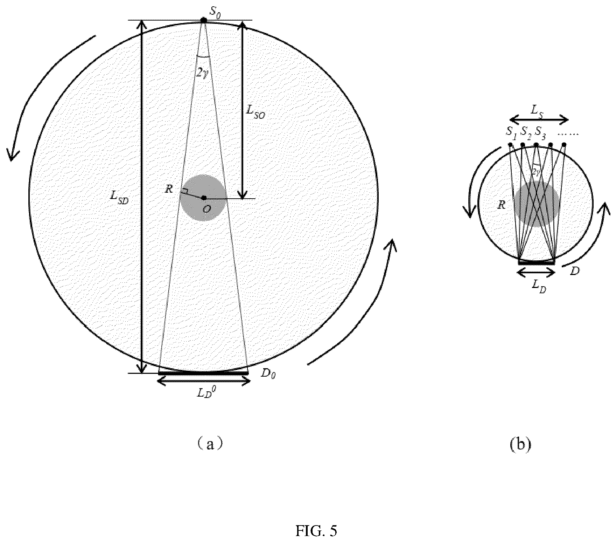

FIG.5 is a comparison schematic diagram showing sizes between a CT scanning system in the prior art and a CT scanning system according to the above embodiments of the present application when the ray divergence angles and imaging fields of view have the same size. Here, it is assumed that the ray divergence angles of the single ray sources of the CT scanning system in the prior art and the CT scanning system of the above embodiments of the present application have the same size, for example, both are $2\gamma$, and it is assumed that the diameters of the imaging fields of view of the two are the same, for example, both are R. From the geometric relationship in FIG. 5, it can be seen that the detector length of the CT scanning system of the prior art in FIG. 5(a) is:

$$L_{D0} = \frac{2 \cdot R \cdot L_{SD}}{\sqrt{L_{SO}^2 - R^2}}$$

The detect scanning system according to the above embodiments of the present application in FIG. 5(b) is (here it is still assumed that the length of the ray source is equal to the detector length):

$$L_D = 2 \cdot R \quad (\text{when } L_S = L_D).$$

From the above two formulas, it can be obtained that

$$\frac{L_D}{L_{D0}} = (2 \cdot R) \Big/ \left( \frac{2 \cdot R \cdot L_{SD}}{\sqrt{L_{SO}{}^2 - R^2}} \right) = \frac{\sqrt{L_{SO}{}^2 - R^2}}{L_{SD}} \ll 1$$

It can be seen that the CT scanning system according to the embodiments of the present application can greatly shorten the detector length compared with the CT scanning system in the prior art.

In addition, in terms of system size, in FIG. 5(a), the distance from the ray source $S_0$ to the rotation center is: $L_{SO} = R/\sin(\gamma)$, without considering the limit of angular distribution, $2\gamma < 90°$, so $L_{SO} > \sqrt{2}R$. However, the CT scanning system according to the embodiments of the present application has no such limitation due to the use of multiple ray sources. Therefore, the CT scanning system according to the embodiments of the present application can minimize the system size.

According to the above comparison, it can be found that the CT scanning system according to the embodiments of the present application can expand the imaging field of view without increasing the overall size of the detector and the system compared with the prior art, so it is particularly suitable for scanning and imaging large-sized objects using high-energy rays. In addition, without expanding the imaging field of view compared with the prior art, the overall size of the detector and the system can be reduced without affecting the image quality.

In addition, in the aforementioned embodiments of the present application, the CT scanning system includes a single ray source group and a single detector corresponding to the single ray source group. However, the present application is not limited to this. According to other embodiments, the CT scanning system may include multiple ray source groups and multiple detectors in one-to-one correspondence with the ray source groups. The ray source group may be a ray source group as described in any of the previous embodiments, and the detector may be a detector as described in any of the previous embodiments, and the specific configuration of each ray source group and each detector may not be repeated. Thus, the CT scanning system including multiple ray source groups and corresponding multiple detector groups can also expand the imaging field of view without increasing the size of the system and the detector, and can reduce the overall size of the detector and the system without affecting the image quality in a case of expanding the imaging field of view compared with the prior art.

The multiple ray source groups may be two or more ray source groups. The multiple ray source groups may be arranged in sequence in the rotation direction, and correspondingly, the multiple detectors may be arranged in sequence in the rotation direction relative to the multiple ray source groups in one-to-one correspondence with each other. In addition, when rotating around the scanned object, the multiple ray source groups and the multiple detector groups rotate synchronously, so that the scanned object can be scanned at multiple rotation angles. Due to the use of multiple ray source groups and corresponding multiple detectors, the CT scanning system according to this embodiment can simultaneously acquire more scanning data at the same rotation angle, thereby improving the imaging speed and improving the quality of the reconstructed image.

The following describes the scanning method of the CT scanning system according to the present application. The CT scanning system according to the embodiments of the present application may also include a control apparatus, which can control at least one ray source group to rotate around the scanned object so that the rays emitted by at least one ray source group cover every point in the scanned object over a range of at least 180 degrees. In the case where the CT scanning system includes only a single ray source group, such as the CT scanning system shown in FIG. 2, the control apparatus can control the ray source group S to rotate around the scanned object (or the rotation center O) so that the rays emitted from the multiple ray sources of the ray source group S can surround every point of the scanned object over a range of at least 180 degrees, thereby obtaining comprehensive scanning data and improving the quality of the reconstructed image.

In addition, during the scanning process, the control apparatus can also control the multiple ray sources of the ray source group S to emit rays in sequence at each rotation angle in a time-sharing on and off manner. Specifically, the control apparatus controls the multiple ray sources of the ray source group S to rotate to the first rotation angle with a predetermined angle amplitude and then stops rotating. At the first rotation angle, the ray source $S_1$ is turned on and the other ray sources are turned off in the first time period so that the ray source $S_1$ emits rays, the ray source $S_1$ and the other ray sources are turned off in the second time period and the ray source $S_2$ is turned on to emit rays, and the ray source $S_2$ and the other ray sources are turned off in the third time period and the ray source $S_3$ is turned on to emit rays; or, the ray source $S_3$ is turned on and the other ray sources are turned off in the first time period so that the ray source $S_3$ emits rays, the ray source $S_3$ and the other ray sources are turned off in the second time period and the ray source $S_2$ is turned on to emit rays, and the ray source $S_2$ and the other ray sources are turned off in the third time period and the ray source $S_4$ is turned on to emit rays. The rest can be done in the same manner until each ray source has experienced ray emission for a predetermined time period, and the control apparatus controls the ray source group S to rotate to the next rotation angle with a predetermined angle amplitude and stops rotating and controls the multiple ray sources of the ray source group S to emit rays in sequence in a similar time-sharing on and off manner at the next rotation angle. The rest can be done in the same manner until the ray source group S rotates by a predetermined rotation angle, such as 180 degrees, and the scanning process is completed.

In the above scanning process, after the control apparatus controls the multiple ray sources of the ray source group S to rotate to a certain rotation angle at a predetermined angle amplitude, the ray source group S stops rotating, and the multiple ray sources of the ray source group S emit rays in sequence to scan the scanned object. However, the scanning process of the present application is not limited to this, and the multiple ray sources of the ray source group S can also emit beams while rotating. For example, the multiple ray sources of the ray source group S can emit beams in sequence at an extremely high speed (for example, pulsed beaming), and the rotation speed of the ray source group S around the scanned object is relatively slow (for example, when the ray source group S is driven to rotate by a slip ring, the rotation speed of the slip ring is relatively slow). In this way, when the ray source group S starts to rotate around the scanned object, multiple ray sources in the ray source group S start to emit beams in sequence at a very fast speed, and the ray source group S only rotates around the scanned object by a very small angle. When all the ray sources of the ray source group S finish emitting beams, the angle change relative to the start of the beam emission is very small, which is equivalent to multiple ray sources emitting ray beams in sequence at the same rotation angle. As a result, there is still a gap between the positions of the multiple ray sources in the ray source group S when they actually emit beams, and it can still ensure that the imaging field of view is expanded relative to the case where only a single ray source emits beams.

In some embodiments, the control apparatus can control the number and position of the ray sources emitting rays according to the size of the scanned object. For example, referring to FIG. 4 (b), when the size of the scanned object is the size of the dark gray area, only the ray source $S_3$ can be controlled to emit rays during the scanning process; when the size of the scanned object is the size of the light gray area, the ray sources $S_1$ to $S_5$ can be controlled to emit rays during the scanning process; when the size of the scanned object is between the above two, only the ray sources $S_1$, $S_3$ and $S_5$ can be controlled to emit rays during the scanning process. In this way, the CT scanning system can adjust the number and position of the ray sources emitting rays in real time according to the size of the scanned object, which can significantly improve the scanning speed and facilitate rapid imaging. In some embodiments, the size of the scanned object can be obtained by, for example, an additional laser scanner, visible light camera, etc., and fed back to the control apparatus. Here, the size of the scanned object can refer to the distance between the two farthest points in the projection of the scanned object in the scanning plane.

When the CT scanning system includes two or more ray source groups, the control apparatus can control the two or more ray source groups to emit rays simultaneously at each rotation angle, and control the multiple ray sources in each ray source group to emit rays in sequence in a time-sharing on and off manner. The specific scanning process is similar to the various scanning processes of controlling multiple ray sources of a single ray source group S to scan as described above, with the only difference being that multiple ray source groups rotate around the scanned object at the same time, and each ray source group emits rays at the same time when the beams are emitted, but the multiple ray sources in each ray source group emit rays in sequence. Since different ray source groups correspond to different detectors, multiple ray source groups emit rays simultaneously, while the ray sources in each group emit rays in sequence, which can improve the scanning efficiency and may not cause data confusion.

The CT scanning system of the embodiments of the present application also includes a data acquisition and processing apparatus, the data acquisition and processing apparatus receives data collected by the detector, integrates information for image reconstruction and generates projection data. Since the same detector receives rays from multiple ray sources corresponding to the ray source group, and the positions of the multiple ray sources are different, and even the ray energies, the divergence angles, etc. may be different, it is necessary to distinguish the data based on the rays of different ray sources. To this end, the data acquisition and processing apparatus of the present application distinguishes data based on rays from different ray sources in the same ray source group by receiving time or using a marking signal. For example, corresponding to the aforementioned scanning process in which multiple ray sources in the ray source group sequentially emit rays in a time-sharing on and off manner, the data acquisition and processing apparatus receives data collected by the detector in a time-sharing acquisition manner. Specifically, at a certain rotation angle, when the first ray source among the multiple ray sources of a ray source group is turned on and emits rays in the first time period, the detector receives the irradiation information of the first ray source, and the data acquisition and processing apparatus receives the data collected by the detector and stores or caches the corresponding projection data and related information together; after the first time period ends, the first ray source is turned off, and the second ray source is turned on and emits ray beams in the second time period, the detector receives the irradiation information of the second ray source, and the data acquisition and processing apparatus receives the data collected by the detector and stores or caches the corresponding projection data and related information together; and so on, until the last ray source in the ray source group is turned on and irradiates for a period of time and the corresponding projection data and related scanning information are stored or cached, the ray source group rotates to the next rotation angle, and the data acquisition and processing apparatus receives the data collected by the detector at the next rotation angle in a similar manner and stores or caches the corresponding projection data and related information, etc., until the ray source group rotates by a predetermined angle, such as 180 degrees, etc. In the case where the CT scanning system includes multiple detectors, the data acquisition and processing apparatus can perform the above operations on the respective detectors at the same time.

In addition, the CT scanning system according to the embodiments of the present application also includes an image analysis and reconstruction apparatus, which processes the projection data generated by the data acquisition and processing apparatus to generate a two-dimensional or three-dimensional image of the scanned object. Specifically, for the same ray source group, since the projection data comes from multiple ray sources and has different geometric parameters (for example, the ray source position, source detection distance, ray vector, etc. are all different), it is necessary to rearrange the projection data from multiple ray sources in the same ray source group before image reconstruction, that is, to weight the projection data according to specific geometric parameters. In addition, there is overlap in the projection data generated by multiple ray sources in the same ray source group. Therefore, the image analysis and reconstruction apparatus also performs redundant processing on the projection data in the overlapping area to maximize the use of the collected data while avoiding image artifacts. In the case where the CT scanning system includes multiple ray source groups, the image analysis and reconstruction apparatus can perform the above operations for the respective ray source groups at the same time.

After data rearrangement and redundant processing, the projection data satisfy the prerequisites for complete reconstruction. The image analysis and reconstruction apparatus can employ analytical reconstruction algorithms, such as a filtered back-projection reconstruction (FBP) algorithm, or iterative reconstruction algorithms, such as an algebraic reconstruction technique (ART), a simultaneous algebraic reconstruction technique (SART), a maximum likelihood estimation (ML), an ordered-subsets expectation maximization (OSEM), etc. to perform an image reconstruction.

The present application also provides a method for scanning a scanned object using a CT scanning system according to any one of the above embodiments, the method comprising: controlling at least one ray source group to rotate around the scanned object so that the rays emitted by at least one ray source group cover every point in the scanned object over a range of at least 180 degrees.

In the case where the CT scanning system includes a single ray source group and a single detector, the method of the present application further comprises controlling multiple ray sources in the ray source group to emit rays in sequence in a time-sharing on and off manner at each rotation angle.

In the case where the CT scanning system includes two or more ray source groups, the method of the present application further comprises controlling two or more ray source groups to emit rays simultaneously at each rotation angle, and controlling multiple ray sources in each ray source group to emit rays in sequence in a time-sharing on and off manner. Since different ray source groups correspond to different detectors, multiple ray source groups emit rays at the same time, and the ray sources in each group emit rays in sequence, which can improve the scanning efficiency and may not cause data confusion.

In addition, the scanning method of the present application also includes receiving data collected by the detector, integrating information for image reconstruction and generating projection data, wherein the data based on the rays of different ray sources in the same ray source group are distinguished by receiving time or using a marking signal, for example, the data collected by the detector is received in a time-sharing acquisition manner. In the case where the CT scanning system includes multiple detectors, the method according to the present application can perform the above operations for the respective detectors at the same time.

In addition, the scanning method of the present application also includes processing the generated projection data to generate a two-dimensional or three-dimensional image of the scanned object, wherein before generating the two-dimensional or three-dimensional image of the scanned object, the image rearrangement is performed on data from the multiple ray sources in the same ray source group and the redundant processing is performed on the overlapping projection data generated by the multiple ray sources in the same ray source group.

In the case where the CT scanning system includes two or more ray source groups, the method according to the present application can perform the above operations for the respective ray source groups at the same time.

The specific implementation of each of the above method steps can refer to the previous description.

The above description of the present application is for the purpose of explanation and description, not for exhausting the present application or limiting the present application to the exact form described. There may be many modifications or variations without departing from the gist of the present application. The scope of the present application is defined by the appended claims.

**Claims**

**1.** A CT scanning system, comprising:

at least one ray source group, configured to emit rays to a scanned object, each of the ray source groups comprising a plurality of ray sources, the plurality of ray sources being arranged at intervals from each other in a same scanning plane and having different ray divergence angle directions; and

at least one detector, arranged in one-to-one correspondence with the at least one ray source group, each detector being configured to receive the rays emitted from the corresponding ray source group and transmitted through the scanned object,

wherein the at least one ray source group is configured to rotate around the scanned object during scanning to scan the scanned object at a plurality of rotation angles, and at the same rotation angle, the plurality of ray sources in each of the at least one ray source group emit ray beams in sequence.

2. The CT scanning system according to claim 1, wherein during scanning, the at least one detector rotates synchronously with the at least one ray source group.

3. The CT scanning system according to claim 1, wherein the ray beams of the plurality of ray sources in each of the ray source groups have a same energy and/or a same divergence angle, or the ray beams of the plurality of ray sources in each of the ray source groups have different energies and/or different divergence angles.

4. The CT scanning system according to claim 1, wherein the energies of the ray beams of the plurality of ray sources in each of the ray source groups are set to decrease in sequence from a middle to both sides of an arrangement of the plurality of ray sources.

5. The CT scanning system according to claim 1, wherein the CT scanning system further comprises a control apparatus configured to control numbers and positions of the ray sources that emit rays in each of the ray source groups according to a size of the scanned object.

6. The CT scanning system according to claim 1, wherein each of the ray source groups is formed as a multi-focus distributed ray source or includes a plurality of independent ray source modules.

7. The CT scanning system according to claim 1, wherein a detector crystal in each of the at least one detector is configured to be independently rotatable, so that a ray receiving surface of the detector crystal in the corresponding detector is configured to be adjusted to be perpendicular to the ray beams of each of the ray sources when the plurality of ray sources in each of the at least one ray source group emit ray beams in sequence.

8. The CT scanning system according to claim 1, further comprising a control apparatus, configured to control the at least one ray source group to rotate around the scanned object so that the rays emitted by the at least one ray source group cover every point in the scanned object over a range of at least 180 degrees.

9. The CT scanning system according to claim 8, wherein the control apparatus is configured to control the plurality of ray sources in each of the at least one ray source group to sequentially emit rays in a time-sharing on and off manner at each of the rotation angles.

10. The CT scanning system according to claim 8, comprising the two or more ray source groups, wherein the control apparatus is configured to control the two or more ray source groups to emit rays simultaneously at each of the rotation angles and control the plurality of ray sources in each of the plurality of ray source groups to sequentially emit rays in a time-sharing on and off manner.

11. The CT scanning system according to any one of claims 1-10, further comprising a data acquisition and processing apparatus, configured to receive data collected by the detector, integrate information for image reconstruction, and generate projection data, wherein the data acquisition and processing apparatus distinguishes data based on rays from different ray sources in the same ray source group by receiving time or using a marking signal.

12. The CT scanning system according to claim 11, wherein the data acquisition and processing apparatus is configured to receive the data collected by the detector in a time-sharing acquisition manner.

13. The CT scanning system according to claim 11, further comprising an image analysis and reconstruction apparatus, configured to process the projection data generated by the data acquisition and processing apparatus to generate a two-dimensional or three-dimensional image of the scanned object, wherein the image analysis and reconstruction apparatus performs an image rearrangement on the data from the plurality of ray sources in the same ray source group and performs a redundant processing on an overlapping projection data generated by the plurality of ray sources in the same ray source group before generating the two-dimensional or three-dimensional image of the scanned object.

14. The CT scanning system according to claim 13, wherein the image analysis and reconstruction apparatus is configured to perform an image reconstruction using an analytical reconstruction algorithm or an iterative reconstruction algorithm.

15. A method for scanning the scanned object using the CT scanning system according to any one of claims 1 to 14, the method comprising:
controlling the at least one ray source group to rotate around the scanned object so that the rays emitted by the at least one ray source group cover every point in the scanned object over the range of at least 180 degrees.

16. The method according to claim 15, further comprising controlling the plurality of ray sources in each of the at least one ray source group to emit rays in sequence in the time-sharing on and off manner at each of the rotation angles.

17. The method according to claim 15, wherein the CT scanning system comprises the two or more ray source groups, and the method further comprises controlling the two or more ray source groups to emit rays simultaneously at each of the rotation angles, and controlling the plurality of ray sources in each of the plurality of ray source groups to emit rays in sequence in the time-sharing on and off manner.

18. The method according to claim 15, further comprising:
receiving the data collected by the detector, integrating the information for image reconstruction, and generating the projection data, wherein the data based on the rays from different ray sources in the same ray source group are distinguished by receiving time or using the marking signal.

19. The method according to claim 18, wherein the data collected by the detector is received in the time-sharing acquisition manner.

20. The method according to claim 18, further comprising processing the generated projection data to generate the two-dimensional or three-dimensional image of the scanned object, wherein before generating the two-dimensional or three-dimensional image of the scanned object, the image rearrangement is performed on data from the plurality of ray sources in the same ray source group and the redundant processing is performed on the overlapping projection data generated by the plurality of ray sources in the same ray source group.

FIG. 1

FIG. 2

(a)                    (b)                    (c)

FIG. 3

(a)                              (b)

FIG. 4

（a）

（b）

FIG. 5

## INTERNATIONAL SEARCH REPORT

| International application No. |
| --- |
| **PCT/CN2023/141574** |

| | |
| --- | --- |
| **A.** | **CLASSIFICATION OF SUBJECT MATTER** |

G01N23/046(2018.01)i; A61B6/03(2006.01)i; G01V5/00(2024.01)i

According to International Patent Classification (IPC) or to both national classification and IPC

| | |
| --- | --- |
| **B.** | **FIELDS SEARCHED** |

Minimum documentation searched (classification system followed by classification symbols)

IPC:G01N A61B G01V

Documentation searched other than minimum documentation to the extent that such documents are included in the fields searched

Electronic data base consulted during the international search (name of data base and, where practicable, search terms used)

CNTXT; ENTXTC; ENTXT; WPABSC; WPABS; CNKI: 清华大学, 同方威视, 发射源, 放射源, 射线, 探测器, 垂直, 分时, 依次, 旋转, 转动, 每个, 多个, 调整, CT, computed tomography, X-ray, source, detector, emit, beam, scan, rotate, angle, multiple

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| PX | CN 116242856 A (TSINGHUA UNIVERSITY et al.) 09 June 2023 (2023-06-09) claims 1-20, and description, paragraphs [0004]-[0085] | 1-20 |
| X | CN 101945614 A (KONINKL PHILIPS ELECTRONICS N.V.) 12 January 2011 (2011-01-12) description, paragraphs [0007]-[0045], and figures 1-3 | 1-20 |
| A | CN 102121908 A (LI TIANFANG et al.) 13 July 2011 (2011-07-13) entire document | 1-20 |
| A | CN 103315761 A (SHENZHEN INSTITUTE OF ADVANCED TECHNOLOGY) 25 September 2013 (2013-09-25) entire document | 1-20 |
| A | CN 106651982 A (XI'AN JIAOTONG UNIVERSITY) 10 May 2017 (2017-05-10) entire document | 1-20 |
| A | CN 107677693 A (NUCTECH CO., LTD. et al.) 09 February 2018 (2018-02-09) entire document | 1-20 |

☐ Further documents are listed in the continuation of Box C.  ☑ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **06 March 2024** | **21 March 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/141574**

| Patent document cited in search report | | | Publication date (day/month/year) | Patent family member(s) | | | Publication date (day/month/year) |
|---|---|---|---|---|---|---|---|
| CN | 116242856 | A | 09 June 2023 | CN | 219915424 | U | 27 October 2023 |
| CN | 101945614 | A | 12 January 2011 | CN | 101945614 | B | 04 December 2013 |
| CN | 102121908 | A | 13 July 2011 | CN | 102121908 | B | 30 October 2013 |
| CN | 103315761 | A | 25 September 2013 | None | | | |
| CN | 106651982 | A | 10 May 2017 | None | | | |
| CN | 107677693 | A | 09 February 2018 | None | | | |

Form PCT/ISA/210 (patent family annex) (July 2022)

**EP 4 628 879 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211708553